# EUROPEAN PATENT SPECIFICATION

(11) **EP 2 582 689 B1**
(45) Date of publication and mention of the grant of the patent: **01.03.2017**
(21) Application number: 11726356.6
(22) Date of filing: 17.06.2011
(51) Int. Cl.: C07D 401/04, A61K 31/505, A61P 35/00

(54) **NEW POLYMORPHIC FORM OF IMATINIB BASE AND PREPARATION OF SALTS THEREOF**
NEUE POLYMORPHE FORM EINER IMATINIB-BASE UND HERSTELLUNG VON SALZEN DARAUS
NOUVELLE FORME POLYMORPHIQUE D'IMATINIB BASE ET PRÉPARATION DE SES SELS

(30) Priority: 22.02.2011 SI 201100054; 19.11.2010 SI 201000398; 27.10.2010 SI 201000348; 18.06.2010 SI 201000181
(43) Date of publication of application: 24.04.2013
(73) Proprietor: KRKA, D.D., Novo Mesto, 8501 Novo mesto (SI)
(72) Inventor: BENKIC, Primoz, 1000 Ljubljana (SI); TIHI, Jaroslav, 8000 Novo mesto (SI); PECAVAR, Anica, 8000 Novo mesto (SI); GERMAN, Tamara, 1000 Ljubljana (SI); VRECER, Franc, 8351 Straza pri Novem mestu (SI); VAJS, Anamarija, 8000 Novo mesto (SI); SKRABANJA, Vida, 8000 Novo mesto (SI)
(74) Representative: Andrae | Westendorp Patentanwälte Partnerschaft
(86) International application number: PCT/EP2011/003025
(87) International publication number: WO 2011/157450

(56) References cited:
- EP-A1- 1 857 454
- WO-A1-2005/077933
- WO-A1-2006/054314
- WO-A1-2008/136010
- WO-A2-2005/095379
- WO-A2-2007/136510
- WO-A2-2008/112722
- WO-A2-2009/151899
- WO-A2-2010/133976

## Description

### FIELD OF THE INVENTION

The invention relates to processes for the preparation of imatinib mesylate form α. Disclosed is a process for the manufacture of new polymorph of imatinib base, the preparation of mesylate salt thereof and their inclusion into pharmaceutical compositions which have an improved stability and purity, as well as processes for their preparation.

### BACKGROUND OF THE INVENTION

Imatinib is the generic name for N-(4-methyl-3-((4-(pyridin-3-yl)pyrimidin-2-yl)amino)phenyl)-4-((4-methylpiperazin-1-yl)methyl)benzamide of the following formula (I):

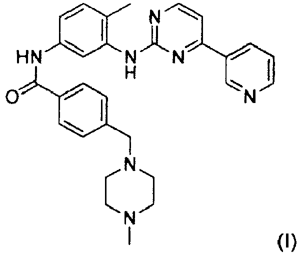

Imatinib is known as a member of agents that act by inhibiting particular tyrosine kinases enzymes and is indicated for the treatment of chronic myeloid leukemia (CML), gastrointestinal stromal tumors (GISTs) and a number of other malignancies. Imatinib may also have a role in the treatment of pulmonary hypertension. The drug is currently being marketed as its monomethanesulfonate salt under the trade names Gleevec® and Glivec® in oral dosage forms such as hard gelatin capsules (100 mg) and coated tablets (100 mg and 400 mg).

The preparation of imatinib and the use thereof, especially in the chemotherapy of tumours, was first disclosed in EP 0564409. However, no solid state characteristics of the base are specified therein.

Imatinib base, as well as the preparation thereof, are further disclosed in many publications. Alternative synthesis paths, isolations and/or purification of imatinib base (column chromatography or crystallization from different solvents) are provided for example in Bioorg. Med. Chem. Lett., 1996, 6: 1221-1226; WO 03/066613; WO 2004/074502; WO 2004/108699; IN 2003MA00462; IN 2003MU01073; US 7507821; WO 2006/071130; IN 2007CH00007; J. Med. Chem., 2005, 48: 249-255; IN 2007CH00620; IN 2007CH00966; WO 2008/057291; WO 2008/059551; Org. Process Res. Dev., 2008, 12: 490-495; Monatsh. Chem., 2009, 140:619-623; WO 2010/014022; WO 2011039782; WO 2011070588.

Crystalline imatinib base form I and the processes for producing form I is provided and characterized by means of XRPD, IR spectrum and DSC curves in patent application EP 1857454 and WO 2008/112722.

In the prior art only one polymorphic form of imatinib base was characterized. Due to an improved stability and solubility in water in comparison to imatinib base, mesylate salt is commercially used.

There is, however, still a need for new imatinib base forms with improved characteristics which can be prepared by processes which are economical, safe and scalable to industrial production. Disclosed is a new polymorphic form of imatinib base which displays improved solubility (particularly over the full physiological pH range 1 to 8), processibility, filterability, and purity. Due to improved characteristics the new crystalline form of base can be converted to pharmaceutically acceptable salts under reaction conditions which are substantially mild, yields and purity of product are high, and consequently the corresponding salt is easily recovered.

The above characteristics are further influenced by the conformation and orientation of molecules in the unit cell defining a particular polymorphic form of a substance. These conformational and orientational factors in turn result in particular intramolecular interactions and intermolecular interactions with adjacent molecules that influence the macroscopic properties of the bulk compound. A particular polymorphic form may give rise to distinct spectroscopic properties that may be detectable by powder X-ray diffraction, solid state 13 C NMR spectrometry and IR spectrometry. The polymorphic form may also give rise to thermal behaviour different from that of the amorphous material or another polymorphic form. Thermal behaviour is measured in the laboratory by such techniques as capillary melting point, thermo gravimetric analysis (TGA) and differential scanning calorimetry (DSC) and may be used for distinguishing polymorphic forms.

The discovery of new polymorphic forms of a pharmaceutically useful compound provides a new opportunity to improve the performance characteristics of a pharmaceutical product. It enlarges the repertoire of materials that a formulation scientist has available for designing, for example, a pharmaceutical dosage form of a drug with a targeted release profile or other desired characteristic.

Imatinib base is the starting material for preparing imatinib salts, especially its monomethanesulfonate salt (mesylate). International application WO 99/03854 discloses two crystalline modifications (α and β form). The invention describes β form which has very advantageous properties in comparison to many advantages related to α form. The exemplified α form is characterized by needle-shaped crystals which are metastable at room temperature and hygroscopic. It is shown that at 25 °C rapidly takes up water so that at 93% relative humidity, the sample is to some extent present in an amorphous form. Because of its physical properties α form is not particularly well suited to pharmaceutical formulation as solid dosage form.

A needle shaped crystal habit usually exhibits a negative influence on the bulk properties of the drug substance, particle size distribution, bulk density, flowability, wetting behavior, surface area and sticking tendency. The poor compression behavior, strong elastic component and high dose of imatinib are other hurdles in the formulation of a stable solid dosage form.

To overcome these drawbacks of the α crystalline form numerous publications provide problem solving methods for stable and processable α crystalline form of imatinib mesylate (WO 2005/095379, WO 2006/024863, WO 2006/048890, WO 2007/136510, WO 2009/151899) as well as novel crystalline forms (δ and ε in WO 2007/023182; α2 in WO 2005/0779333; I and II in WO 2006/054314; H1 in WO 2004/0106326; IV, V, VI, VII, VIII, IX, X, XI, XII, XIII, XIV, XV and XVI in WO 2007/136510).

WO 2010/133976 A2 comprises Examples concerned with the preparation of α-Form of Imatinib mesylate.

Anhydrous amorphous forms are for example disclosed in US 2009181977 and WO 2009/147626. Imatinib mesylate solvates are also known in the prior art.

Most of the disclosed processes are performed at relatively high temperatures in the process of preparing imatinib mesylate. Thus, there still exists a need for processes at lower temperature to overcome formation of undesired impurities, degradation and reaction by-products. The presence of impurities like methyl methanesulfonate and ethylmethanesulfonate in imatinib mesylate which may affect adversely has been disclosed in J. Pharm. Biomed. Anal., 2008, 46: 780-783.

Various other acid addition salts of imatinib have been disclosed. In particular, hydrated or anhydrous halogenated salts were described in WO 2004/074502. For example tartrate, such as a (D)(-) tartrate or a (L)(+) tartrate, hydrochloride, citrate, malate, fumarate, succinate, benzoate, benzenesulfonate, pamoate, formate, malonate, 1,5-naphthalenedisulfonate, salicylate, cyclohexanesulfamate, lactate, mandelate, hemiglutarate, adipate, squarate, vanillate, oxaloacetate, ascorbate and sulfate salts are known from WO 2005/075454. In WO 2009/065910 a further group of acid addition salts of imatinib consisting of orotate, oxalate, nicotinate, camphor sulfonate, para-toluene sulfonate, laurate, aspartate, caprate, oleate and phenyl acetate salt were introduced.

Thus, the specific physical and chemical properties of imatinib mesylate, especially the α crystalline form, and the high doses have been known to cause problems in the preparation of formulations suitable for an effective oral administration. Hence, a need exists for formulations of imatinib mesylate for effective oral delivery, which formulations provide for an appropriate disintegration time and/or appropriate solubility and/or dissolution profile of the active principle. In addition, it is desirable to be able to prepare such formulations by using an economical process which avoids the problems of the prior art as noted above. Such a highly pure active ingredient and process for its preparation at mild conditions introduced by present invention ensures product quality and minimizes the safety risk to the patient with a need to be treated with imatinib mesylate belonging to a group of oncology products.

### FIGURES

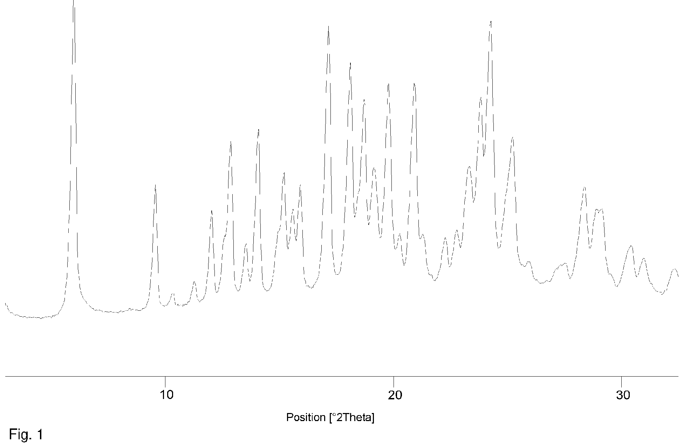

- Figure 1: shows an XRPD spectrum of imatinib base polymorphic form I.
- Figure 2: shows an XRPD spectrum of imatinib base polymorphic form II.
- Figure 3: shows an IR spectrum of imatinib base polymorphic form II.
- Figure 4: shows DSC curve of imatinib base polymorphic form II.
- Figure 5: shows spherical shaped crystalline form α of imatinib mesylate.
- Figure 6: shows primary particles of crystalline form α of imatinib mesylate after milling.

### DETAILED DESCRIPTION OF THE INVENTION

According to an aspect, there is provided a process of conversion of any form of imatinib mesylate to imatinib mesylate form α, as defined in claim 1 annexed.

According to a further aspect, there is provided a process for the preparation of imatinib mesylate form α, as defined in claim 3 annexed.

According to a first embodiment polymorphic form II of imatinib base is provided.

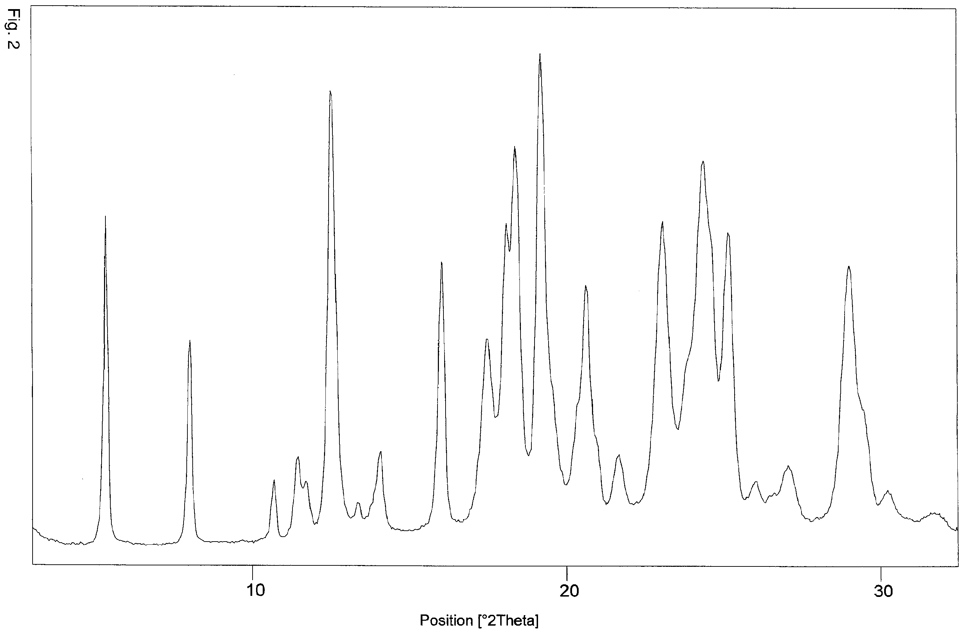

Polymorphic form II is characterized by a X-ray powder diffraction pattern exhibiting characteristic diffractions (diffraction peaks) at about 5,4; 8,0; 12,6; 16,1; 17,5; 19,3; 20,7; 23,1; 24,4 and 29,1, each ± 0,2 degrees 2-theta. Preferably, form II is characterized by an X-ray powder diffraction pattern exhibiting characteristic diffractions as outlined in Figure 2.

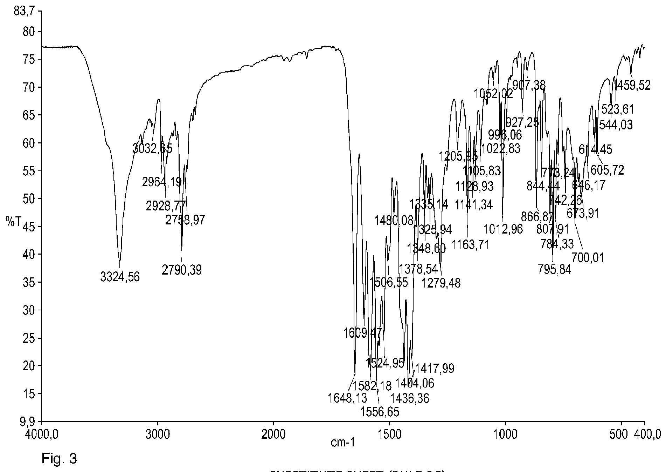

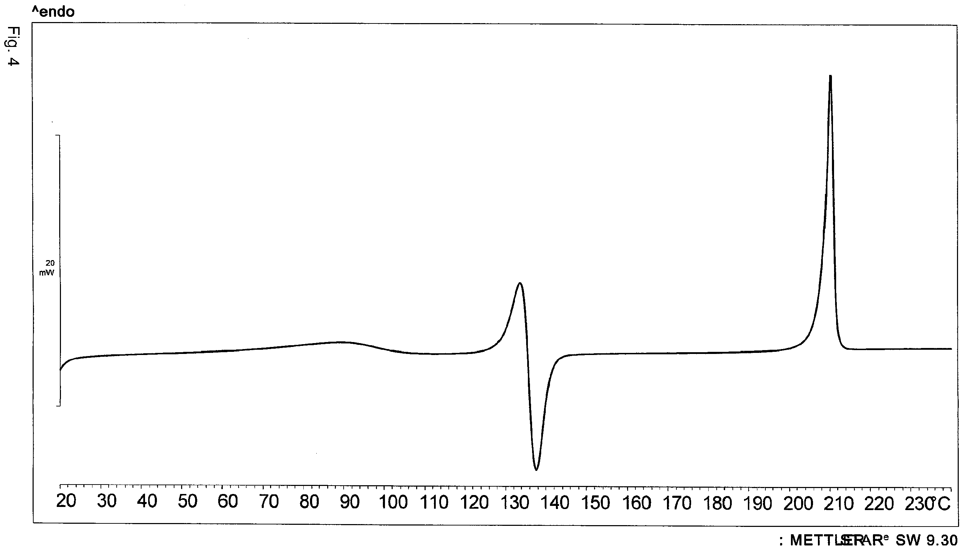

Polymorphic form II is characterized by IR spectrum and by DSC curve as outlined in Figures 3 and 4, respectively.

Polymorphic form II is characterized by a endothermic peak at 130 °C as outlined by DSC curve in figure 4.

Polymorphic form II is characterized by laser diffraction, for instance using a Malvern Mastersizer instrument using isopar L as the dilution medium (A=0,01) for particle size distribution. The average particle size could be in a range 1-200 µm more preferably 5-100 µm.

Disclosed is a polymorphic form of imanitib base which is obtainable by the processes as described herein.

Imatinib base may be also present in form of hydrates or solvates.

In prior art knowledge no efficient and robust procedure for isolating the imatinib base in the solid and pure form is provided. We surprisingly found out that pure imatinib base can be easily isolated from a reaction mixture or recovered from imatinib acid addition salts. Preferably, a water mixture with alcohols is used as a solvent and preferably the pH is varied in the range between 7-11. So obtained solid imatinib base has good filtration properties, can be obtained in high yield and purity and exhibits spectra as shown in Figures 2-4.

Disclosed is a process for the preparation of imatinib base polymorphic form II, The process comprises the steps of:
(i) combining imatinib salt, imatinib base or reaction mixture comprising imatinib base or imatinib salts with water or with a mixture of water and alcohol C1-C4,
(ii) precipitating of base by addition of alkaline substance to adjust the pH of the suspension to a pH value between 7-11, preferably between 8-9, most preferably to 8.5,
(iii) isolating the precipitated base and drying the product at temperatures between 30 °C and 120 °C, more preferably between 40 °C and 80 °C, most preferably between 50 °C and 70 °C.

The vol./vol. concentration of the alcohol in the mixture of water and alcohol ranges from 1-50 %, preferably 2-30 %, more preferably 5-20 %, most preferably 5-15 %.

Alcohols C1 to C4, i.e. alcohols with 1 to 4 carbon atoms (also designated C1 to C4 alcohols herein), can be e.g. selected from the group of: methanol, ethanol, n-propanol, 2-propanol, n-butanol, preferably ethanol or 2-propanol, more preferably 2-propanol.

Solute /solvent ratio can e.g. vary between 1:5 to 1:50, preferably 1:10 to 1:30, more preferably 1:20 to 1:25, most preferably 1:15 to 1:25.

Alkaline substance can e.g. be selected from: inorganic bases like alkali metal hydroxides, alkali metal carbonates, alkaline earth metal hydroxides, alkali earth metal carbonates, metal hydroxides, aqueous ammonia, ammonia gas, liquid ammonia or organic bases like alkyl amines, aryl amines and their amine derivatives, more preferably ammonia and alkali metal hydroxides, most preferably aqueous ammonia and/or sodium hydroxide.

The product can be isolated by any conventional method known in the art selected without limitations, for example filtration, centrifugation, decantation.

Drying as used herein refers to the removal of solvent residues performed for example by heating at normal pressure or in vacuum, where the material is kept in stationary phase or material is continuously moving, e.g. fluid bed drying.

Imatinib base as used in item (i) above and in the further embodiments below can be prepared by any conventional method known to the skilled person. For example, imatinib base can be prepared according to methods as described in Bioorg. Med. Chem. Lett., 1996, 6: 1221-1226; WO 03/066613; WO 2004/074502; WO 2004/108699; IN 2003MA00462; IN 2003MU01073; US 7507821; WO 2006/071130; IN 2007CH00007; J. Med. Chem., 2005, 48: 249-255; IN 2007CH00620; IN 2007CH00966; WO 2008/057291; WO 2008/059551; Org. Process Res. Dev., 2008, 12: 490-495; Monatsh. Chem., 2009, 140:619-623; WO 2010/014022; WO 2011039782; or WO 2011070588.

Any imatinib base can be recrystallized by any known techniques in the art, where imatinib base polymorph II can optionally be used as seeding material.

Polymorphic form II of imatinib base could be used for the preparation of suitable salts such as mesylate, dimesylate, tartrate, such as a (D)(-) tartrate or a (L)(+) tartrate, hydrochloride, citrate, malate, fumarate, succinate, benzoate, benzenesulfonate, pamoate, formate, malonate, 1,5-naphthalenedisulfonate, salicylate, cyclohexanesulfamate, lactate, mandelate, hemiglutarate, adipate, squarate, vanillate, oxaloacetate, ascorbate, sulfate, orotate, oxalate, nicotinate, camphor sulfonate, para-toluene sulfonate, laurate, aspartate, caprate, oleate and phenyl acetate salt. Said salts may be obtained for example by adding corresponding organic or inorganic acid to the polymorphic form II of imatinib base in a manner well known to the skilled person.

Imatinib base in polymorphic form II is especially advantageous for preparation of imatinib mesylate form α according to processes described in present application.

We surprisingly found that polymorph II of imatinib base is kinetic polymorph of imatinib base as compared to polymorph I according to EP 1857454 and that it shows better solubility and dissolution rates compared to polymorph I.

Since the solubility and dissolution rates of the polymorphic form II are better than in previously disclosed form, imatinib base can be converted to imatinib salts at mild conditions, specially at lower temperatures. Therefore subjecting the active ingredient to elevated temperatures which may result in formation of undesired impurities, degradation and reaction by-products, for example as disclosed in Callis et al., Risk Assessment of Genotoxic Impurities in Marketed Compound Administered over a Short-Term Duration: Applications to Oncology Products and Implications for Impurity Control Limits, Org. Process Res. Dev., web published in June 2010 is avoided.

In a further (second) embodiment of the invention preparation of
imatinib mesylate at low temperatures is provided.

The process comprises the steps of:
(i) combining imatinib base with water and/or a mixture of water with alcohols C1-C4 and 0.9 to 1.2 equivalent of organic or inorganic acid, said organic or inorganic acid being methanesulfonic acid,
(ii) stirring the reaction mixture at low temperatures of 5 °C to 70 °C, more preferably at 5 °C to 50 °C, most preferably at 10 °C to 30 °C until a clear solution is obtained; optionally the obtained solution is filtered before further use,
(iii) crystallizing imatinib salt by addition of or combining with organic solvent inoculated with seeding crystals and stirring the reaction mixture, preferably until crystallization of imatinib mesylate form α is completed,
(iv) isolating the polymorphic form α of imatinib mesylate and drying the product at temperatures between 30 °C and 100 °C, preferably between 50 °C and 70 °C.

Alcohols C1 to C4 can e.g. be selected from the group of: methanol, ethanol, n-propanol, 2-propanol, n-butanol, preferably ethanol or 2-propanol, more preferably 2-propanol.

A mixture of water with C1 to C4 alcohols may comprise 0.1 to 99.9 % water, preferably 0.1 to 50 % water, most preferably 0.1 to 25 % water.

Organic solvent defined under item (iii) can e.g. be selected from a group of higher ketones, esters, alcohols, diethylcarbonate. Preferable solvents are methyl isobutyl ketone, ethylacetate, ethanol, 2-propanol, n-butanol, most preferable are 2-propanol or methyl isobutyl ketone. Mixtures of organic solvents can also be used. The volume to weight ratio (V/w) of organic solvent per mass of imatinib salt in reaction can e.g. range between 1:5 to 1:50, preferably 1:10 to 1:30, more preferably 1:15 to 1:25.

The term "equivalent" means normalized molar ratio with respect to stoichiometric ratio between base and salt formation acid in particular salt. For example 1.0 equivalent of acid addition salt in formation of imatinib mesylate means that 1 mole of imatinib base is reacted with 1 mole of methanesulfonic acid. During the synthesis of imatinib dimesylate salt 1.0 equivalent of acid addition salt means, that 1 mole of the imatinib base is reacted with 2 moles of methanesulfonic acid.

According to the above described process under item (i) imatinib base in any polymorphic form or mixture thereof can be used, preferably polymorphic form II is converted to the imatinib mesylate.

According to the present invention highly pure imatinib mesylate is obtained with HPLC purity more than 99 %, preferably 99.5 %, most preferably 99.9 % and with low content of degradation and reaction by-products related to processes performed at elevated temperatures.

The product obtained according to the second embodiment of the invention can be used in process for preparation of pharmaceutical formulation, with the advantage of low bulk volume, advantageous ratio between tapped and bulk density, good compressibility as well as low chargeability and hygroscopicity.

In a further (third) embodiment of the invention a process of conversion of any form of imatinib mesylate to imatinib mesylate form α at low temperatures is provided.

The process comprises the steps of:
(i) combining imatinib mesylate with water and/or a mixture of water with alcohols C1-C4; optionally 0.01-10 %, preferably 0.01-5% molar excess of methanesulfonic acid with respect to imatinib salt can be added,
(ii) stirring the reaction mixture at low temperatures of 5 °C to 70 °C, more preferably at 5 °C to 50 °C, most preferably at 10 °C to 30 °C a until clear solution is obtained; optionally the obtained solution is filtered before further use,
(iii) crystallizing imatinib mesylate by addition of or combining with organic solvent inoculated with imatinib mesylate α crystals and stirring the reaction mixture, preferably until crystallization of imatinib mesylate form α is completed,
(iv) isolating the polymorphic form α of imatinib mesylate and drying the product at temperatures between 30 °C and 100 °C, preferably between 50 °C and 70 °C.

Disclosed is that instead of methanesulfonic acid defined under item (i) tartaric such as (D)(-) tartaric or (L)(+) tartaric, hydrochloric, citric, malic, fumaric, succinic, benzoic, benzenesulfonic, pamoic, formic, malonic, 1,5-naphthalenedisulfonic, salicylic, cyclohexanesulfamic, lactic, mandelic, hemiglutaric, adipic, squaric, vanillic, oxaloacetic, ascorbic, sulfuric, orotic, oxalic, nicotinic, camphor sulfonic, para-toluene sulfonic, lauric, aspartic, capric, oleic and phenyl acetic acid or any other pharmaceutically acceptable salt formation acid can be used with respect to imatinib salt that is crystallized.

Alcohols C1 to C4 can be e.g. selected from the group of: methanol, ethanol, n-propanol, 2-propanol, n-butanol, preferably ethanol or 2-propanol, more preferably 2-propanol.

The mixture of water with C1 to C4 alcohols may comprise 0.1 to 99.9 % water, preferably 0.1 to 50 % water, most preferably 0.1 to 25 % water.

The organic solvent mentioned in step (iii) is e.g. selected from a group of higher ketones, esters, alcoholes, diethylcarbonate, preferable solvents are methyl isobutyl ketone, ethylacetate, ethanol, 2-propanol, n-butanol, most preferable are 2-propanol or methyl isobutyl ketone. The volume to weight ratio (V/w) of organic solvent per mass of imatinib salt in reaction can e.g. range between 1:5 to 1:50, preferably 1:10 to 1:30, more preferably 1:15 to 1:25.

According to the above described process under item (i) imatinib mesylate in any polymorphic form or mixture thereof, amorphous form, hydrates, solvates can be used to prepare imatinib mesylate α form with advantageous properties for pharmaceutical process and stability.

The product so obtained exhibits same beneficial properties obtained by the second embodiment.

Imatinib mesylate polymorphic form α used as a seeding material in the processes according to second and third embodiments can be prepared by any of methods known in the state of the art such as: WO 99/03854, WO 2005/095379, WO 2006/024863, WO 2006/048890, WO 2007/136510, WO 2009/151899.

According to a further (fourth) embodiment a process for preparation of imatinib mesylate form α without seeding procedure and at low temperatures is provided.

The process comprises the steps of:
(i) combining or dissolving imatinib base and organic solvent,
(ii) adding 0.9 to 1.2 equivalent of methanesulfonic acid or other salt formation acid, most preferably 1 to 1.05 equivalent at temperatures 5 °C to 70 °C, more preferably 5 °C to 50 °C, most preferably 10 to 30 °C,
(iii) stirring the solution or suspension, preferably until crystallization of imatinib salt, preferably imatinib mesylate form α is completed,
(iv) isolating the imatinib salt, preferably polymorphic form α of imatinib mesylate and drying the product at temperatures between 30 °C and 100 °C, more preferably between 50 °C and 70 °C.

The organic solvent under item (i) can e.g. be selected from a group of esters, ketones or alcohols, more preferably ethylacetate, 2-propanol and ethanol. Organic solvent can comprise 0 % to 25 % water, preferably 0 to 10 % water, most preferable 0% to 5 % water.

By salt formation acids defined under item (ii) for example methanesulfonic, tartaric such as (D)(-) tartaric or (L)(+) tartaric, hydrochloric, citric, malic, fumaric, succinic, benzoic, benzenesulfonic, pamoic, formic, malonic, 1,5-naphthalenedisulfonic, salicylate, cyclohexanesulfamic, lactic, mandelic, hemiglutaric, adipic, squaric, vanillic, oxaloacetic, ascorbic, sulfuric, orotic, oxalic, nicotinic, camphor sulfonic, para-toluene sulfonic, lauric, aspartic, capric, oleic and phenyl acetic acid or any other pharmaceutically acceptable salt formation acid is meant. Mixtures of salt formation acids can also be used.

The term "equivalent" means normalized molar ratio with respect to stechiometric ration between base and salt formation acid in particular salt. For example 1.0 equivalent of acid adition salt in formation of imatinib mesylate means that 1 mole of imatinib base is reacted with 1 mole of methanesulfonic acid. During the synthesis of imatinib dimesylate salt 1.0 equivalent of acid addition salt means, that 1 mole of the imatinib base is reacted with 2 moles of methanesulfonic acid.

According to the above described process under item (i) imatinib base in any polymorphic form or mixture thereof can be used, preferably polymorphic form II is converted to the imatinib mesylate.

According to the present invention highly pure imatinib mesylate is obtained with HPLC purity more than 99 %, preferably 99.5 %, most preferably 99.9 % and with low content of degradation and reaction by-products related to processes performed at elevated temperatures.

The product obtained according to the fourth embodiment can be used in process for preparation of pharmaceutical formulation, with the advantage of low bulk volume, advantageous ratio between tapped and bulk density, good compressibility as well as low chargeability and hygroscopicity.

With the processes described in the embodiments disclosed herein, very pure imatinib mesylate is obtained, especially with respect to 6-Methyl-*N*¹-(4-(pyridin-3-yl)pyrimidin-2-yl)benzene-1,3-diamine of the following formula (II) that is known reagent used for imatinib base synthesis. The impurity (Formula II) is in the present invention named as I-1.

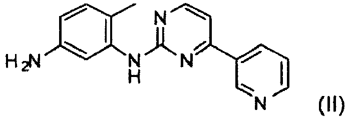

### Formula II: Impurity I-1

With processes disclosed in the second, third and fourth embodiments assay of impurity I-1 in imatinib base can be reduced for more than 5 times, preferably more than 10 times, more preferably more than 20 times and most preferably more than 30 times, in particular with high yields. Various salts can be crystallized according to disclosed salt formation processes to achieve required purifications. Preferably imatinib mesylate and most preferably imatinib dimesylate is crystallized. Imatinib salts prepared according to the second, third and fourth embodiment can be further transformed to very pure imatinib base as described for example in the first embodiment. Such base can be further transformed into imatinib mesylate form α, with morphologic characteristics, which are especially advantageous in the drying step of the imatinib mesylate α form. Imatinib mesylate form α with assay of residual organic solvents below 2 %, preferably below 1 % and most preferably below 0.5 % can be easily obtained.

According to an aspect of the invention, it has been found that drying of imatinib mesylate α form is specially efficient if crystallization of imatinib salt, in particular as described in step (iii) according to second and third embodiment of present invention, is performed at temperatures between 25 and 75 °C, preferably between 30 and 70 °C and most preferably between 50 and 60 °C.

Also, when methanesulfonic acid is used as salt formation acid alkyl mesylates are kept below 1.6 ppm, preferably below limit of detection according to GC-MS method disclosed in the present invention. Even more, final imatinib mesylate prepared according to the present invention contains less than 100 ppm of impurity I-1, preferably less than 10 ppm, more preferably less than 5 ppm, most preferably less than 2 ppm, as determined by disclosed LC-MS method.

According to a further (fifth) embodiment, the obtained product with advantageous properties can be directly formulated into solid pharmaceutical composition that means without a need for grinding or milling in order to reduce particle size specially the length of needle like crystals of imatinib or its salts.

The said solid pharmaceutical composition is preferably in the form of tablets, pills, powders, lozenges, sachets, soft and hard gelatine capsules, suppositories etc. The dosage form is preferably suitable for oral application.

The present pharmaceutical formulations can be prepared by known technological procedures, e.g. by direct compression, dry, wet or melt granulation including other agglomeration procedures where active substance according to the present invention is agglomerated together with at least one inactive pharmaceutically acceptable substance such as an excipient having melting and/or softening point below 170 °C, preferably below 150 °C and most preferably below 120 °C, using well known and readily available excipients. Specially preferred are the processes which exclude water or at which water is present in low amounts or concentrations, typically less than 10 vol/w % of water calculated on the weight of dry components of solid composition is used in the manufacturing procedure of solid compositions with imatinib or its pharmaceutically acceptable salts. In the preparation of the compositions of imatinib mesylate, the active ingredient will usually be mixed with an excipient or mixture of excipients, or diluted with an excipient or mixture of excipients. In a special alternative of the fifth embodiment imatinib or its salt can be agglomerated by state of the art processes where no or only small amount of water is used in the manufacturing process.

The solid pharmaceutical compositions in addition to the active ingredient can further comprise one or more pharmaceutically acceptable carriers. Suitable carriers can optionally be selected from the group consisting of but not limited to a diluent, a binder, a disintegrant, a lubricant and/or glidant. Optionally, the solid pharmaceutical composition can be further coated.

The diluent can be selected from the group consisting of but not limited to microcrystalline cellulose, powdered cellulose, composite materials combining crystalline cellulose with lactose (Cellactose, Tablettose), guar gum (Avicel CE15) or silicified cellulose (Prosolv), corn starch, maize starch, starch derivatives such as pregelatinized starch, calcium hydrogen phosphate in anhydrous and hydrated form, various types of sugars such as lactose (anhydrous and monohydrate), compressible sugar, fructose, dextrates, sugar alcohols such as mannitol, sorbitol, maltitol, xylitol, lactitol, or other sugars such as saccharose, raffinose, trehalose, fructose or mixture thereof, calcium carbonate, calcium lactate or mixture thereof.

The binder can be selected from the group consisting of but not limited to polyvinylpyrrolidone, microcrystalline cellulose, cellulose ether, hydroxyethylcellulose, hydroxypropylcellulose, hydroxypropylmethylcellulose of different grades (i.e. viscosity), corn starch, maize starch, pregelatinised starch, polymethacrylate, or mixtures thereof.

The disintegrant can be selected from the group consisting of but not limited to crospovidone, starch, starch derivatives such as pregelatinised starch and sodium starch glycollate, microcrystalline cellulose, carboxymethylcellulose sodium (CMC-Na) or calcium (CMC-Ca), cross-linked CMC-Na, polacrilin potassium, low-substituted hydroxypropylcellulose or mixtures thereof.

The lubricant can be selected from the group consisting of but not limited to stearic acid, magnesium stearate, magnesium palmitate, magnesium oleate, magnesium lauryl sulfate, hydrogenated vegetable oil, hydrogenated castor oil, talc, sodium stearyl fumarate, glyceryl behanate, macrogols or mixtures.

The glidant can be selected from the group consisting of but not limited to colloidal silicon dioxide, talc, stearic acid, palmitic acid, polyethylene glycol, carnauba wax or mixtures thereof.

Optionally, cores/tablets can be coated with conventional materials used for film coating, i.e. as described in Pharmaceutical Coating Technology (G. Cole (ed.), 1995). Film coating formulations usually contain the following components:
- polymer(s),
- plasticizer(s),
- colourant(s)/opacifier(s),
- vehicle(s).

In film coating suspension additional quantities of flavours, surfactants, adhesion enhancers such as for example saccharides selected from but not limited to the group consisting of polydextrose, maltodextirn and lactose and waxes can be used. The majority of the polymers used in film coating can be either cellulose derivatives, such as the cellulose ethers, or acrylic polymers and copolymers. Occasionally encountered can be high molecular weight polyethylene glycols, polyvinyl pyrrolidone, polyvinyl alcohol and waxy materials. Their function usually is to prevent bad mouth feel and/or taste and in some cases degradation, e.g. oxidation of the active ingredients and/or excipients used.

It has been surprisingly found that a stable and effective pharmaceutical solid composition with high or low drug (active ingredient) load can be prepared from active ingredient obtained according to the present invention and/or by the processes and compositions according to the present invention. By high active ingredient load it is meant that an active ingredient, i.e. imatinib in the form of pharmaceutically acceptable salts, preferably mesylate, is present in the composition in concentration of more than 60 w/w%, preferably of more than 70 w/w% and most preferably of more than 80 w/w% calculated on the total weight of solid composition.

In low active ingredient load compositions, preferably tablets, imatinib or imatinib in the form of pharmaceutically acceptable salts, preferably mesylate, is present in the concentration of less than 30 w/w% calculated on the total weight of solid composition, preferably in the range of 25 w/w% to 30 w/w%, more preferably in the range of 26 w/w% to 28 w/w%.

Optionally, the active ingredient can be grinded or milled in order to reduce particle size, especially the length of needle like crystals of imatinib or its salts.

In a first alternative of the fifth embodiment, agglomeration processes can e.g. be selected from dry granulation processes such as slugging and/or roller compaction. Imatinib or its salts is mixed with at least one excipient selected from diluent, binder, disintegrator, surfactant, lubricant, glidant and compressed into brikets or compactants, which are then crushed, optionally sieved, mixed with optional extragranular excipients and compressed into tablets or filled into capsules or sachets.

In a second alternative of the fifth embodiment agglomeration can be performed by wet granulation using organic solvents or a mixture of one or more water miscible organic solvents and water where the ratio of mass of dry substances and water is more than 10 to 1, preferably of more than 25 to 1.

In a third alternative of the fifth embodiment agglomeration can be performed by mixing of active substance and at least one excipient selected from diluent, binder, desintegrant, lubricant, glidant, surfactant, where at least one of the selected excipients is melted or softened and mixed with the rest of ingredients. In still another embodiment active ingredient in powder form is mixed the one or more excipients selected from diluent, binder, desintegrant, lubricant, glidant, surfactant, where at least one excipient melts or is softened during mixing by external heating or due to the friction and/or pressure during mixing. The melted and/or softened excipient serves as agglomeration liquid, which binds powder particles into agglomerates.

Agglomerates obtained by the second and third alternatives of the fifth embodiment can optionally be sieved or in case the particle size of agglomerates is too big, they can be milled. Optionally milled and/or sieved agglomerated (granulated) material can be filled directly into capsules or into sachets or can be mixed in appropriate mixer such as conical, biconical, cubic or low or high share mixers with one or more pharmaceutically acceptable excipients selected from binder, disintegrant, diluent, glidant, lubricant and compressed into tablets preferably of round or oval or capsule like shape with maximal length or diameter of less than 18 mm, preferably of less than 15 mm with tablet weight of e.g. less than 1,000 mg, preferably of less than 800 mg. The weight of tablets with low active ingredient load is e.g. less than 1,500 mg.

It has been found that the solid oral dosage form which is preferably prepared by the process of wet granulation is stable at ICH stability testing conditions. Further, the preparation of particles of granulate consisting of imatinib mesylate and excipient(s), provides a reproducible and processible formulation.

The granulate obtained can be optionally compressed into tablets with prior addition of extragranular excipients or filled into capsules. For capsule filling e.g. granulate only or granulate with addition of talc can be used. Therefore very high amount, e.g. more than 80% of imatinib mesylate in weight based on total weight of capsule content can be used.

The said dosage form is stable at ICH stability conditions, with a stable imatinib mesylate α form and is not hygroscopic.

The invention is illustrated by reference to the following examples. However, the examples are not intended to limit any scope of the claim anyway. It will be apparent to those skilled in the art that many modifications, both to materials and methods, may be practiced without departing from the purpose and interest of this invention.

### PREPARATIVE EXAMPLES

### Analytical methods:

Particle size distribution has been determined by laser diffraction, for instance a Malvern Mastersizer instrument using isopar L as the dilution medium (A=0,01).

X-ray powder diffraction patterns were obtained by Phillips PW3040/60 X'Pert PRO powder diffractometer; X-celerator detector at CuKα radiation 1,541874Å, 3°<2θ<31°.

DSC scans were recorded on DSC 822e Mettler Toledo scanning calorimeter. Samples of approx. 3 mg were scanned between 20°C and 250°C at heating rate of 10°C/min under nitrogen atmosphere.

The process control and product purity is assessed by high pressure liquid chromatography (HPLC) with a column of the type X Bridge C-8 75 x 4.6mm, 2.5µm; detector: UV240 nm; flow rate: 0.7 ml/min; injection volume: 5 µl; mobile phase: A: 0.01M (NH₄)₂HPO₄ pH=6.0; B: methanol (v/v); Gradient: 0'=20%B, 15'-18'=80%B, 19'-21'= 90%B, 22'=20%B. This HPLC method is generally applicable for the analysis of imatinib, chromatographic purity and process control.

### Detailed description:

### HPLC method for chromatographic purity and related substances

| | |
|---|---|
| Column: | X Bridge C-18 75 x 4.6mm, 2.5µm, |
| Eluent A: | 0.01 M (NH₄)₂HPO₄ pH=6.0 |
| Eluent B: | methanol |

**Gradient:**

| Time (min) | Eluent A (%) | Eluent B (%) |
|---|---|---|
| 0 | 80 | 20 |
| 15 | 20 | 80 |
| 18 | 20 | 80 |
| 19 | 10 | 90 |
| 21 | 10 | 90 |
| 22 | 80 | 20 |

| | |
|---|---|
| Post time: | 3 min |
| Flow-rate: | 0.7 ml/min |
| Detection: | UV, wavelength 240 nm |
| Injection volume: | 5 µl |
| Column temperature: | 25 °C |
| Diluent: | acetonitrile : water (30 : 70, V:V) |

### LC-MS method for assay of I-1

| | |
|---|---|
| Column: | X-Bridge SHIELD RP18 100 x 2.1mm; 3.5µm |
| Mobile phase: | |

Gradient elution
Eluent A: 0.1 % formic acid in water
Eluent B: acetonitrile : methanol = 3 : 7 (VN)

| Time (min) | Eluent A (%) | Eluent B (%) |
|---|---|---|
| 0.00 | 80 | 20 |
| 3.00 | 80 | 20 |
| 3.10 | 50 | 50 |
| 7.00 | 50 | 50 |
| 7.10 | 80 | 20 |
| 10.00 | 80 | 20 |

| | |
|---|---|
| Flow rate: | 0.3 ml/min |
| T _{of sample}: | 15°C |
| Column temperature: | 30 °C |
| Injection: | 3 µl |

**Mass detection conditions:**

| | |
|---|---|
| Ionization: | ESP ^{⊕} |
| Detection mode: | SRM for I-1 |

1.) 278.2 > 106.2
2.) 278.2 > 262.2

### GC/MS method for assay of alkyl mesylates

**Chromatographic conditions:**

| | |
|---|---|
| Column: | Capillary, ZB FFAP (30 m x 0.32 mm, film thickness 0.25 mm) |
| Carrier gas: | helium |
| Velocity: | 1 ml/min |
| Split ratio: | 1 : 1 |
| Column temperature: | 100 °C (30 min) |
| Injector temperature: | 250 °C |
| AUX temperature: | 260 °C |
| Injection: | 2.0 ml |

**Mass detection conditions:**

| | |
|---|---|
| Ionization: | EI |
| Detection mode: | SIM of 109, 95, 80 for methyl mesylate |
| | SIM of 109, 97, 79 for ethyl mesylate |
| | SIM of 123, 97, 79 for isopropyl mesylate |

### Preparative example 1

3 g of imatinib base is supended in 210 ml of 2-propanol. The suspension is warmed up to reflux temperature. Solution is filtered and 0.414 ml methanesulfonic acid is added. The solution is heated at reflux temperature for another five minutes and cooled to about room temperature in two hours and further stirred for at least three hours. Imatinib mesylate is filtered off, washed with 2-propanol and vacuum dried at 50 °C.
3.11 g of imatinib mesylate, α form is obtained (yield 87 %).

### Preparative example 2

In 35.5 ml of ethanol abs., 1.5 g of imatinib base is supended. Suspension is warmed up to reflux temperature and 0.183 ml of methanesulfonic acid is added. Hot solution is filtered and stirred overnight at room temperature. Imatinib mesylate is filtered off and dried in vacuum dryer at 40 °C.
1.38 g of imatinib mesylate (77 % yield), α form is obtained.

### Preparative example 3

Imatinib base, 1.0 g is suspended in 30 ml of ethyl acetate and suspension is warmed up to reflux temperature. 0.138 ml of methanesulfonic acid is added, stirred at reflux temperature for about 30 minutes and cooled to room temperature. Suspension is stirred overnight and product is filtered off and washed with ethyl acetate and vacuum dried at 50 °C.
0.98 g of imatinib mesylate (82 % yield), α form is obtained.

### Preparative example 4

In 1.21 l of water 55 g of imatinib mesylate is dissolved. 8.25 ml of 25 % ammonia solution is slowly added. The oily suspension is stirred overnight and filtered off and washed with water. Obtained imatinib base is vacuum dried at 50 °C.
44.7 g of imatinib base is obtained with 97 % yield.

### EXAMPLES

### Preparation of imatinib base polymorphic form II:

### Example 1.1

In 44 ml of water 2 g of imatinib mesylate is dissolved. 0.3 ml of 25 % ammonia solution is added until pH 8.5 is obtained. The oily suspension is warmed up to 40 °C and 4 ml of ethanol is added. The oily suspension is crystallized and suspension is stirred at room temperature for two hours. The product is filtered off, washed with water and vacuum dried at 50 °C.
1.5 g of imatinib base is obtained with 90 % yield.

### Example 1.2

Imatinib mesylate (80.0 g) is suspended in 1600 ml of water and stirred until whole substance is dissolved. To the clear solution of imatinib mesylate 160 ml of 2-propanol is added and pH correction of imatinib mesylate solution is performed with ammonia aqueous solution until pH 8.5 is reached. The suspension is stirred overnight, filtered off, dried at 55 °C until the temperature of the substance is the same as temperature of heating media.
Yield: 94.2 %

### Example 1.3

In 1.1 l of water and 100 ml of 2-propanol 50 g of imatinib mesylate is dissolved. 7.5 ml of 25 % ammonia solution is added until pH 8.5 is obtained. Suspension is further stirred overnight and filtered off and washed with water. Obtained imatinib base is vacuum dried at 70 °C.
40 g of imatinib base is obtained with 95 % yield.

### Example 1.4

Imatinib base (5.0 g) is suspended in 100 ml water. Imatinib base is dissolved with adding of hydrochloric acid. Complete dissolving of base is reached at pH 4.04. To clear solution of imatinib base 10 ml of 2-propanol is added and pH of solution is continuously corrected with ammonia aqueous solution to 8.5. The suspension stirred overnight, the product is filtered off, washed with water and dried at 55 °C until the temperature of the substance is the same as temperature of heating media.
Yield: 93.2 %

### Preparation of imatinib mesylate polymorphic form α

### Example 2.1

0.5 g of imatinib base is suspended in 35 ml 2-propanol and 0.065 ml of methanesulfonic acid is added at room temperature. The suspension is stirred overnight, imatinib mesylate is filtered off, washed with 2-propanol and vacuum dried at 50 °C.
0.59 g of imatinib mesylate (89 % yield), α form is obtained.

### Example 2.2

2.0 g of imatinib base is dissolved in 10 ml of water and 0.26 ml of methanesulfonic acid. After all substance is dissolved, 9 ml of water is distilled off (vacuum distillation). To the residue at room temperature 50 ml 2-propanol which contains 20 mg of imatinib mesylate α form is added in one portion. Obtained suspension is stirred overnight at room temperature. The product is filtered off, washed with 2-propanol and vacuum dried at 50 °C.
2.3 g of imatinib mesylate (96.6 % yield) α form is obtained.

### Example 2.3

In mixture 35 ml of water and 0.966 ml of methanesulfonic acid 7.0 g of imatinib base is dissolved at 50 °C. Solution is filtered and 29 ml of water is distilled off. To the oil residue at 40 °C 210 ml of 2-propanol, contains 70 mg of imatinib mesylate α form is added in one portion. The suspension is cooled to room temperature and stirred overnight.
The product is filtered off, washed with 2-propanol and vacuum dried at 50 °C. 7.87 g of imatinib mesylate (93 % yield) α form is obtained.

### Example 2.4

In the mixture of 3.5 ml water, 10.5 ml 2-propanol and 0.966 ml of methanesulfonic acid 7.0 g of imatinib base is dissolved at 50 °C. The solution is filtered and and filter is washed with the mixture of 1 ml of water and 4 ml of 2-propanol. The solution is cooled to 30 °C and 140 ml of 2-propanol and 70 mg of imatinib mesylate α form is added in one portion. The suspension is cooled to room temperature and stirred overnight. The product is filtered off, washed with 2-propanol and vacuum dried at 50 °C.
7.55 α of imatinib mesylate (90 % yield) α form is obtained. Content of residual 2-propanol is 1.9 %.

### Example 2.5

3.8 g of imatinib base (with content of impurity I-1 181.8 ppm) is dissolved in 12.0 ml of mixture of 2-propanol and water (3.5 ml of water and 10.5 ml of 2-propanol) and 0.50 ml of methanesulfonic acid (purity < 99.5 %) at room temperature. After imatinib base is completely dissolved solution is filtered and filter plate is washed with 1.5 ml of mixture of 2-propanol and water (3.5 ml of water and 10.5 ml of 2-propanol). Clear solution is added in three minutes in reaction vessel equipped with mechanical stirrer to 120.0 ml of 2-propanol and 38 mg of imatinib mesylate form α. Obtained suspension is stirred for four hours, product is filtered off, washed with 10 ml of 2-propanol and vacuum dried at 70 °C for 15 hours.

Weight of dry product (imatinib mesylate) is 4.2 g (93 % yield) and content of impurity I-1 is 13.25 ppm, chromatographic purity is 99.8 %, single impurity is less than 0.10 %.

### Example 2.6

10.0 g of imatinib base (with content of impurity I-1 566 ppm) is dissolved in 31.4 ml of mixture of 2-propanol and water (9.0 ml of water and 27.0 ml of 2-propanol) and 2.632 ml of methanesulfonic acid (purity < 99.5 %) at room temperature. After imatinib base is completely dissolved solution is filtered and filter plate is washed with 2.0 ml of mixture of 2-propanol and water (9.0 ml of water and 27.0 ml of 2-propanol). In reaction vessel equipped with mechanical stirrer in three minutes clear solution is added to 314.0 ml of 2-propanol. Obtained suspension is stirred overnight and product is filtered off, washed with 2-propanol and vacuum dried at 50 °C for 15 hours.

Weight of dry product (imatinib dimesylate) is 12.633 g (91 % yield) and content of impurity I-1 is 21.4 ppm.

7.0 g of imatinib dimesylate (content of impurity I-1 is 21.4 ppm) is dissolved in 140.0 ml water. After imatinib dimesylate is completely dissolved 14.0 ml of 2-propanol is added. With diluted ammonia solution pH is corrected to 8.5. At pH 7.6 solutions becomes turbid and at pH 8.5 it becomes crystalline. Suspension is stirred at room temperature for 4 hours and pH of suspension is continuously corrected to 8.5. After four hours product imatinib base is filtered off, washed with water and vacuum dried at 50 °C for five hours. Weight of dry product (imatinib base) is 4.6 g (93 % yield).

3.5 g of imatinib base (with content of impurity I-1 26.2 ppm) is dissolved in 11.0 ml of mixture of 2-propanol and water (3.5 ml of water and 10.5 ml of 2-propanol) and 0.461 ml of methanesulfonic acid (purity < 99.5 %) at room temperature. After imatinib base is completely dissolved, solution is filtered. In reaction vessel equipped with mechanical stirrer in three minutes clear solution is added to 110.0 ml of 2-propanol and 35 mg of imatinib mesylate form α. Filter plate is washed with 1.4 ml of mixture of 2-propanol and water (3.5 ml of water and 10.5 ml of 2-propanol). Obtained suspension is stirred for four hours. Product (imatinib mesylate) is filtered off, washed with 2-propanol and vacuum dried at 70 °C for 15 hours.
Weight of dry product (imatinib mesylate) is 3.8 (91 % yield) and content of impurity I-1 is 2.7 ppm, alkyl mesylates are below 1.6 ppm according to the GC-MS method. Chromatographic purity is 99.9 %, single impurity is below 0.10 %.

### Example 2.7

10.2 g of imatinib base is dissolved in 32.0 ml of mixture of 2-propanol and water (9.0 ml of water and 27.0 ml of 2-propanol) and 1.342 ml of methanesulfonic acid (purity < 99.5 %) at room temperature. After imatinib base is completely dissolved solution is filtered and filter plate is washed with 4.0 ml of mixture of 2-propanol and water (9.0 ml of water and 27.0 ml of 2-propanol). Clear solution is warmed up to 45 °C and added in three minutes in reaction vessel equipped with mechanical stirrer to 320.0 ml of 2-propanol and 100 mg of imatinib mesylate form α at temperature 45 °C. Obtained suspension is stirred for three hours at 45 °C. Product is filtered off, washed with 2-propanol and vacuum dried at 70 °C for 15 hours.
Weight of dry product (imatinib mesylate) is 10.68 g (88 % yield) and content of 2-propanol is 4300 ppm. Bulk volume is 5.3 ml/g.

### Example 2.8

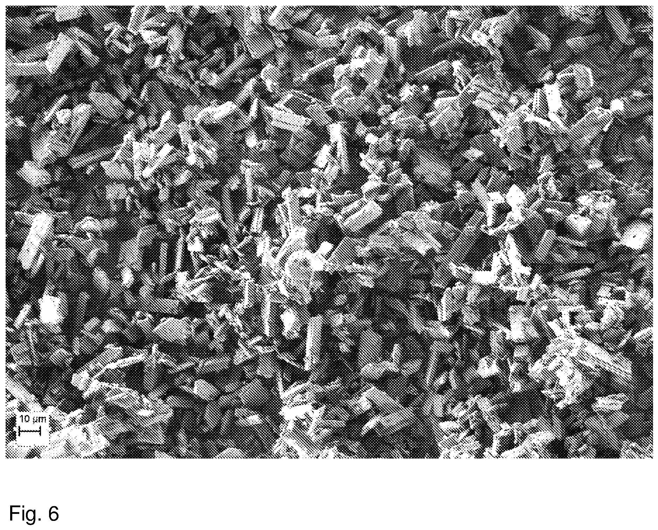

16 g of imatinib base (content of impurity I-1 is 35.8 ppm) is dissolved in mixture of 29.8 ml of 2-propanol and 10.9 ml of water and 3.14 ml of methanesulfonic acid (purity < 99 %) at temperature up to 45 °C. After imatinib base is completely dissolved solution is filtered and filter plate is washed with mixture of 2-propanol (3.8 ml) and water (1.9 ml). Clear solution is added in 3.5 minutes in reaction vessel equipped with mechanical stirrer to suspension of 383 ml of 2-propanol (preheated to 58 °C) and 160 mg of imatinib mesylate form α. Temperature of suspension is kept in the range 55 °C to 65 °C for two hours. After two hours the suspension is gradually cooled to room temperature and at room temperature is further stirred for two hours. The product is filtered off, washed with 20 ml of 2-propanol and vacuum dried at 80 °C for 15 hours. Dried material is milled. Primary particles after milling are shown in Fig. 6.

Weight of dry product (imatinib mesylate) is 17.2 g (90.3 % yields) and content of I-1 impurity is 4.2 ppm. Chromatographic purity is 99.8 %, content of single impurity is less than 0.10 %. Content of 2-propanol is 3837 ppm.

### Example 2.9

16 g of imatinib base (content of impurity I-1 is 35.8 ppm) is dissolved in mixture of 41.3 ml of 2-propanol and 13.8 ml of water and 3.14 ml of methanesulfonic acid (purity < 99 %) at temperature up to 45 °C. After imatinib base is completely dissolved solution is filtered and filter plate is washed with mixture of 2-propanol (1.7) and water (0.6 ml). Clear solution is added in 4 minutes in reaction vessel equipped with mechanical stirrer to suspension of 503 ml of 2-propanol (preheated to 58 °C) and 160 mg of imatinib mesylate form α. Temperature of suspension is kept in the range 55 °C to 65 °C for two hours. After two hours the suspension is gradually cooled to room temperature and at room temperature is further stirred for two hours. The product is filtered off, washed with 20 ml of 2-propanol and vacuum dried at 70 °C for 15 hours.

Weight of dry product (imatinib mesylate) is 17.0 g (88.9 % yields) and content of I-1 impurity is 2.6 ppm. Chromatographic purity is 99.9 %, content of single impurity is less than 0.10 %. Content of 2-propanol is 4125 ppm.

### Recrystalisation of imatinib mesylate of any polymorphic form to imatinib mesylate of form α

### Example 3.1

0.9 g of imatinib mesylate in polymorphic form β was dissolved in 0.9 ml of water and 0.9 ml of 2-propanol at room temperature. Clear solution is filtered glassfibre filtre and washed with 0.5 ml of mixture 2-propanol:water 1:1. To the solution 18 ml of 2-propanol and 9 mg of imatinib mesylate α is added in one portion. The suspension is stirred for 3 hours, and polymorphic form α of solid is confirmed by IR spectroscopy. Suspension is stirred overnight. The product is filtered off, washed with 2-propanol and vacuum dried at 50 °C.
0.8 g of imatinib mesylate (90 % yield) α form is obtained.

### Example 3.2

8 g of imatinib mesylate was dissolved in 4 ml of water and 12 ml of 2-propanol at 40 °C. Solution was cooled to 25 °C and 150 ml of 2-propanol and 80 mg of imatinib mesylate α was added to the solution. Suspension was stirred at 25 °C for 3 hours. The product is filtered off, washed with 2-propanol and vacuum dried at 30 °C to 40 °C for 2 hour and at 50 °C for 5 hours.
7.45 g of imatinib mesylate (93 % yield) α form is obtained. Content of residual 2-propanol is 1.8 %.

### Example 3.3

1 g of imatinib mesylate was dissolved in 12 ml of 96% ethanol in water at 70 °C temperature. To the clear solution 17 ml of methyl isobutyl ketone and 10 mg of imatinib mesylate α is added. Suspension was cooled to room temperature and stirred at room temperature for 4 hours. The product is filtered off, washed with 2-propanol and vacuum dried at 50 °C for 5 hours.
0.86 g of imatinib mesylate (86 % yield) α form is obtained.

### Example 3.4

8 g of imatinib mesylate was dissolved in 4 ml of water and 12 ml of 2-propanol at 40 °C. Solution was cooled to 25 °C and 150 ml of 2-propanol and 80 mg of imatinib mesylate α was added to the solution. Suspension was stirred at 25 °C for 2 hour, then heated to 30 °C and 66 ml of solvent is distilled off (vaccum destilation). The product was stirred 15 hours, filtered off, washed with 2-propanol and vacuum dried at 30 °C to 40 °C for 2 hour and at 50 °C for 5 hours.
7.37 g of imatinib mesylate (92 % yield) α form is obtained.

### Example 3.5

6 g of imatinib mesylate (content of I-1 is 62 ppm) was dissolved in 3.95 ml of water and 11.8 ml of 2-propanol at 40 °C. 50 µg of methanesulfonic acid with assay 99.5% (representing 5 % excess of methanesulfonic acid with respect to dissolved imatinib mesylate) was added to the solution. Solution was cooled to 25 °C and filtered into 188 ml of 2-propanol previously inoculated with 60 mg of imatinib mesylate α. Suspension was stirred at 25 °C for 5 hour, filtered off, washed with 2-propanol and vacuum dried at 30 °C to 40 °C for 2 hour and at 70 °C for 10 hours.
5.37 g of imatinib mesylate (89 % yield) α form is obtained with I-1 impurity 6.5 ppm.

### Example 3.6

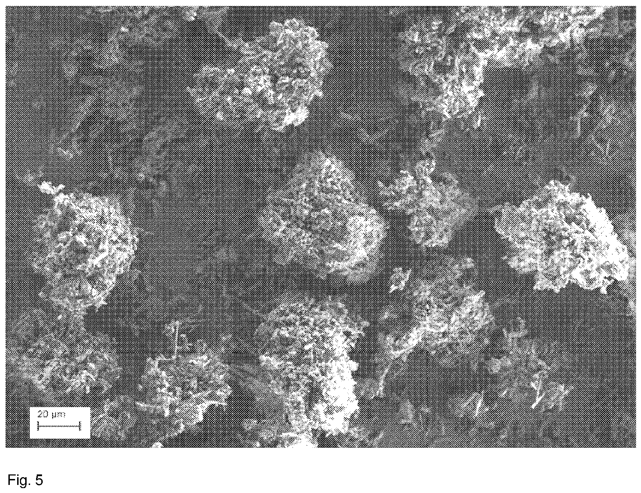

Imatinib mesylate (30 g) was dissolved in mixture of 20 ml water and 59 ml 2-propanol at temperature 40 °C and cooled to 25 °C. In 2L reactor equipped with overhead stirrer 790 ml of 2-propanol was maintained at 5 °C. 250 mg of imatinib of form α was suspended in those 790 ml of 2-propanol by ultraturrax to obtain homogene suspension. Solution with imatinib mesylate was filtered in so prepared suspension at 5 °C in one portion, so that temperature in reactor is raised to about 10 °C. Suspension was cooled to 5 °C and crystallized for 20 hours. Then product was filtered and washed with 200 ml of 2-propanol under nitrogen atmosphere. Product was dried in vaccum dryer at 70 °C for 15 hours, then product was slowly allowed to cool in vacuum until room temperature. 25.3 g of imatinib mesylate form α was obtained with morphology shown in Fig. 5. Residual 2-propanol in final product was 1800 ppm. Bulk volume of material is 3.3 ml/g.

### Example 3.7

6 g of imatinib mesylate (content of impurity I-1 is 6.1 ppm) is dissolved in mixture of 13.5 ml of 2-propanol and 4.5 ml of water at temperature up to 45 °C. After imatinib mesylate is completely dissolved solution is filtered. Clear solution is added in 2 minutes in reaction vessel equipped with mechanical stirrer to suspension of 160 ml of 2-propanol (preheated to 58 °C) and 160 mg of imatinib mesylate form α. Temperature of suspension is kept in the range 55 °C to 65 °C for two hours. After two hours the suspension is gradually cooled to room temperature and at room temperature is further stirred for two hours. The product is filtered off, washed with 20 ml of 2-propanol and vacuum dried at 80 °C for 15 hours.
Weight of dry product (imatinib mesylate) is 5.3 g (88.3 % yields). Chromatographic purity is 99.8 %, content of single impurity is less than 0.10 %. Content of 2-propanol is 3541 ppm and content of impurity I-1 is 1.8 ppm.

### Example 3.8

6 g of imatinib mesylate is dissolved in mixture of 10.5 ml of 2-propanol and 4 ml of water at temperature up to 45 °C. After imatinib mesylate is completely dissolved solution is filtered. Clear solution is added in 2 minutes into reaction vessel equipped with mechanical stirrer to suspension of 118 ml of 2-propanol (preheated to 58 °C) and 160 mg of imatinib mesylate form α. Temperature of suspension is kept in the range 55 °C to 65 °C for two hours. After two hours the suspension is gradually cooled to room temperature and at room temperature is further stirred for two hours. The product is filtered off, washed with 20 ml of 2-propanol and vacuum dried at 80 °C for 15 hours.
Weight of dry product (imatinib mesylate) is 5.5 g (91.3 % yields). Chromatographic purity is 99.8 %, content of single impurity is less than 0.10 %. Content of 2-propanol is 3880 ppm.

### Pharmaceutical compositions

### Example 4.1

| | |
|---|---|
| Imatinib mesylate | 119.5 mg |
| Mannitol | 26.0 mg |
| Hydroxypropylcellulose | 2.0 mg |
| Crospovidone | 15.0 mg |
| Mannitol | 22.35 mg |
| Magnesium stearate | 1.25 mg |
| Colloidal Anhydrous Silica | 1.40 mg |
| Isopropyl Alcohol | Qs |

Imatinib mesylate and mannitol were mixed in a high shear granulator. The solution of hydroxypropylcellulose in isopropyl alcohol was added to the mixture to obtain a granulate. After drying and sieving of the granulate crospovidone, mannitol, magnesium stearate and colloidal anhydrous silica were added to obtain the compression mixture. After compression, the obtained tablets were optionally film coated with ready to make film coating suspension. Theoretical weight of the film coated tablets was 196 mg.

### Example 4.2

| | |
|---|---|
| Imatinib mesylate | 119.5 mg |
| Mannitol | 26.0 mg |
| Hydroxypropylcellulose | 2.0 mg |
| Crospovidone | 28.0 mg |
| Mannitol | 9.35 mg |
| Magnesium stearate | 1.25 mg |
| Colloidal Anhydrous Silica | 1.40 mg |
| Isopropyl Alcohol | Qs |

Imatinib mesylate and mannitol were mixed in a high shear granulator. The solution of hydroxypropylcellulose in isopropyl alcohol was added to the mixture to obtain a granulate. After drying and sieving of the granulate crospovidone, mannitol, magnesium stearate and colloidal anhydrous silica were added to obtain the compression mixture. After compression, the obtained tablets were optionally film coated with ready to make film coating suspension. Theoretical weight of the film coated tablets was 196 mg.

### Example 4.3

| | |
|---|---|
| Imatinib mesylate | 119.5 mg |
| Mannitol | 26.0 mg |
| Hydroxypropylcellulose | 2.0 mg |
| Croscarmellose sodium | 4.5 mg |
| Mannitol | 32.85 mg |
| Magnesium stearate | 1.25 mg |
| Colloidal Anhydrous Silica | 1.40 mg |
| Isopropyl Alcohol | Qs |

Imatinib mesylate and mannitol were mixed in a high shear granulator. The solution of hydroxypropylcellulose in isopropyl alcohol was added to the mixture to obtain a granulate. After drying and sieving of the granulate croscarmellose sodium, mannitol, magnesium stearate and colloidal anhydrous silica were added to obtain the compression mixture. After compression, the obtained tablets were optionally film coated with ready to make film coating suspension. Theoretical weight of the film coated tablets was 196 mg.

### Example 4.4

| | |
|---|---|
| Imatinib mesylate | 59.75 mg |
| Mannitol | 13.0 mg |
| Hydroxypropylcellulose | 1.0 mg |
| Talc | 0.75 mg |
| Isopropyl Alcohol | Qs |

Imatinib mesylate and mannitol were mixed in a high shear granulator. The solution of hydroxypropylcellulose in isopropyl alcohol was added to the mixture to obtain a granulate. After drying and sieving of the granulate talc was added. The obtained mixture was filled into capsules.

### Example 4.5

| | |
|---|---|
| Imatinib mesylate | 119.5 mg |
| Mannitol | 26.0 mg |
| Hydroxypropylcellulose | 2.0 mg |
| Talc | 1.50 mg |
| Isopropyl Alcohol | Qs |

Imatinib mesylate and mannitol were mixed in a high shear granulator. The solution of hydroxypropylcellulose in isopropyl alcohol was added to the mixture to obtain a granulate. After drying and sieving of the granulate talc was added. The obtained mixture was filled into capsules.

### Example 4.6

| | |
|---|---|
| Imatinib mesylate | 239.0 mg |
| Mannitol | 52.0 mg |
| Hydroxypropylcellulose | 4.0 mg |
| Talc | 3.0 mg |
| Isopropyl Alcohol | Qs |

Imatinib mesylate and mannitol were mixed in a high shear granulator. The solution of hydroxypropylcellulose in isopropyl alcohol was added to the mixture to obtain a granulate. After drying and sieving of the granulate talc was added. The obtained mixture was filled into capsules.

### Example 4.7

| | |
|---|---|
| Imatinib mesylate | 478.0 mg |
| Mannitol | 104.0 mg |
| Hydroxypropylcellulose | 8.0 mg |
| Talc | 6.0 mg |
| Isopropyl Alcohol | qs |

Imatinib mesylate and mannitol were mixed in a high shear granulator. The solution of hydroxypropylcellulose in isopropyl alcohol was added to the mixture to obtain a granulate. After drying and sieving of the granulate talc was added. The obtained mixture was filled into capsules.

### Example 4.8

| | |
|---|---|
| Imatinib mesylate | 119.5 mg |
| Mannitol | 26.0 mg |
| Sodium alginate | 3.0 mg |
| Crospovidone | 15.0 mg |
| Mannitol | 21.35 mg |
| Magnesium stearate | 1.25 mg |
| Colloidal Anhydrous Silica | 1.40 mg |
| Isopropyl Alcohol | qs |

Imatinib mesylate, mannitol and sodium alginate were mixed in a high shear granulator. Isopropyl alcohol was added to the mixture to obtain granulate. After drying and sieving of the granulate crospovidone, mannitol, magnesium stearate and colloidal anhydrous silica were added to obtain the compression mixture. After compression, the obtained tablets were optionally film coated with ready to make film coating suspension. Theoretical weight of the film coated tablets was 193 mg.

### Example 4.9

| | |
|---|---|
| Imatinib mesylate | 119.5 mg |
| Mannitol | 26.0 mg |
| Sodium alginate | 3.0 mg |
| Croscarmellose sodium | 15.0 mg |
| Mannitol | 21.35 mg |
| Magnesium stearate | 1.25 mg |
| Colloidal Anhydrous Silica | 1.40 mg |
| Isopropyl Alcohol | qs |

Imatinib mesylate, mannitol and sodium alginate were mixed in a high shear granulator. Isopropyl alcohol was added to the mixture to obtain a granulate. After drying and sieving of the granulate croscarmellose sodium, mannitol, magnesium stearate and colloidal anhydrous silica were added to obtain the compression mixture. After compression, the obtained tablets were optionally film coated with ready to make film coating suspension. Theoretical weight of the film coated tablets was 193 mg.

### Example 4.10

| | |
|---|---|
| Imatinib mesylate | 119.5 mg |
| Mannitol | 26.0 mg |
| Carbomer | 1.5 mg |
| Crospovidone | 15.0 mg |
| Mannitol | 22.85 mg |
| Magnesium stearate | 1.25 mg |
| Colloidal Anhydrous Silica | 1.40 mg |
| Isopropyl Alcohol | qs |

Imatinib mesylate, mannitol and carbomer were mixed in a high shear granulator. Isopropyl alcohol was added to the mixture to obtain a granulate. After drying and sieving of the granulate crospovidone, mannitol, magnesium stearate and colloidal anhydrous silica were added to obtain the compression mixture. After compression, the obtained tablets were optionally film coated with ready to make film coating suspension. Theoretical weight of the film coated tablets was 193 mg.

### Example 4.11

| | |
|---|---|
| Imatinib mesylate | 119.5 mg |
| Mannitol | 94.0 mg |
| Low-substituted hydroxypropyl cellulose LH-21 | 40.0 mg |
| Colloidal Anhydrous Silica | 1.0 mg |
| Purified water | q.s. |
| Microcrystalline cellulose | 60.0 mg |
| | |
| Starch, low moisture | 26.0 mg |
| Crospovidone | 15.0 mg |
| Colloidal Anhydrous Silica | 1.2 mg |
| Magnesium stearate | 3.3 mg |
| Ready to make coating suspension | q.s. |

Imatinib mesylate, colloidal anhydrous silica and mannitol were mixed and granulated with solution of purified water and low substituted hydroxypropyl cellulose on a fluid bed granulator. After drying and sieving of the granulate crospovidone, microcrystalline cellulose, low moisture starch, magnesium stearate and colloidal anhydrous silica were added to obtain the compression mixture. After compression, the obtained tablets were optionally film coated with ready to make film coating suspension. Theoretical weight of the film coated tablets was 370 mg.

### Example 4.12

| | |
|---|---|
| Imatinib mesylate | 119.5 mg |
| Mannitol | 94.0 mg |
| Low-substituted hydroxypropyl cellulose LH-21 | 40.0 mg |
| Colloidal Anhydrous Silica | 1.0 mg |
| Isopropyl alcohol | q.s. |
| Microcrystalline cellulose | 60.0 mg |
| | |
| Starch, low moisture | 26.0 mg |
| Crospovidone | 15.0 mg |
| Colloidal Anhydrous Silica | 1.2 mg |
| Magnesium stearate | 3.3 mg |
| Ready to make coating suspension | q.s. |

Imatinib mesylate, colloidal anhydrous silica and mannitol were mixed and granulated with solution of isopropyl alcohol and low substituted hydroxypropyl cellulose on a fluid bed granulator. After drying and sieving of the granulate crospovidone, microcrystalline cellulose, low moisture starch, magnesium stearate and colloidal anhydrous silica were added to obtain the compression mixture. After compression, the obtained tablets were optionally film coated with ready to make film coating suspension. Theoretical weight of the film coated tablets was 370 mg.

### Example 4.13

| | |
|---|---|
| Imatinib mesylate | 119.5 mg |
| Lactose monohydrate | 120.0 mg |
| Starch | 20.0 mg |
| Colloidal Anhydrous Silica | 1.0 mg |
| Hydroxypropylcellulose | 10.0 mg |
| Purified water | q.s. |
| Microcrystalline cellulose | 60.0 mg |
| Crospovidone | 25.0 mg |
| Colloidal Anhydrous Silica | 1.2 mg |
| Magnesium stearate | 3.3 mg |
| Ready to make coating suspension | q.s. |

Imatinib mesylate, colloidal anhydrous silica, lactose and starch were mixed and granulated with solution of purified water and hydroxypropyl cellulose on a fluid bed granulator. After drying and sieving of the granulate crospovidone, microcrystalline cellulose, magnesium stearate and colloidal anhydrous silica were added to obtain the compression mixture. After compression, the obtained tablets were optionally film coated with ready to make film coating suspension. Theoretical weight of the film coated tablets was 370 mg.

### Example 4.14

| | |
|---|---|
| Imatinib mesylate | 119.5 mg |
| Lactose monohydrate | 120.0 mg |
| Starch | 20.0 mg |
| Colloidal Anhydrous Silica | 1.0 mg |
| Hydroxypropylcellulose | 10.0 mg |
| Isopropyl alcohol | q.s. |
| Microcrystalline cellulose | 60.0 mg |
| Crospovidone | 25.0 mg |
| Colloidal Anhydrous Silica | 1.2 mg |
| Magnesium stearate | 3.3 mg |
| Ready to make coating suspension | q.s. |

Imatinib mesylate, colloidal anhydrous silica, lactose and starch were mixed and granulated with solution of isopropyl alcohol and hydroxypropyl cellulose on a fluid bed granulator. After drying and sieving of the granulate crospovidone, microcrystalline cellulose, magnesium stearate and colloidal anhydrous silica were added to obtain the compression mixture. After compression, the obtained tablets were optionally film coated with ready to make film coating suspension. Theoretical weight of the film coated tablets was 370 mg.

### Example 4.15

| | |
|---|---|
| Imatinib mesylate | 119.5 mg |
| Mannitol | 120.0 mg |
| Hydroxypropylcellulose | 5.0 mg |
| Colloidal Anhydrous Silica | 1.0 mg |
| Isopropyl alcohol | q.s. |
| | |
| Mannitol | 85.0 mg |
| | |
| Crospovidone | 25.0 mg |
| Colloidal Anhydrous Silica | 1.2 mg |
| Magnesium stearate | 3.3 mg |
| Ready to make coating suspension | q.s. |

Imatinib mesylate, colloidal anhydrous silica and mannitol were mixed and granulated with solution of isopropyl alcohol and hydroxypropyl cellulose on a fluid bed granulator. After drying and sieving of the granulate crospovidone, mannitol, magnesium stearate and colloidal anhydrous silica were added to obtain the compression mixture. After compression, the obtained tablets were optionally film coated with ready to make film coating suspension. Theoretical weight of the film coated tablets was 370 mg.

### Example 4.16

| | |
|---|---|
| Imatinib mesylate | 119.5 mg |
| Mannitol | 120.0 mg |
| Hydroxypropylcellulose | 5.0 mg |
| Colloidal Anhydrous Silica | 1.0 mg |
| Purified water | q.s. |
| | |
| Mannitol | 85.0 mg |
| | |
| Crospovidone | 25.0 mg |
| Colloidal Anhydrous Silica | 1.2 mg |
| Magnesium stearate | 3.3 mg |
| Ready to make coating suspension | q.s. |

Imatinib mesylate, colloidal anhydrous silica and mannitol were mixed and granulated with solution of purified water and hydroxypropyl cellulose on a fluid bed granulator. After drying and sieving of the granulate crospovidone, mannitol, magnesium stearate and colloidal anhydrous silica were added to obtain the compression mixture. After compression, the obtained tablets were optionally film coated with ready to make film coating suspension. Theoretical weight of the film coated tablets was 370 mg.

### Example 4.17

**Tablet core**

| | |
|---|---|
| Imatinib mesylate | 119.5 mg |
| Mannitol | 8.5 mg |
| Crospovidone | 8.0 mg |
| Magnesium stearate | 1.0 mg |
| Colloidal Anhydrous Silica | 1.0 mg |
| Weight of tablet core | 138.0 mg |
| *Composition of ready to make coating suspension* | qs |
| Polivynyl alcohol | 40.0 % w/w |
| Titanium dioxide | 25.0 % w/w |
| Macrogol 3000 | 20.2 % w/w |
| Talc | 14.8 % w/w |

Imatinib mesylate and mannitol were mixed and dry granulated on a compactor. After sieving of the granulate crospovidone, magnesium stearate and colloidal anhydrous silica were added to obtain the compression mixture. After compression, the obtained tablets were optionally film coated with ready to make film coating suspension (until the tablet weight gain of 2.9%). Theoretical weight of the film coated tablets was 142 mg.

### Example 4.18

| | |
|---|---|
| Imatinib mesylate | 119.5 mg |
| Mannitol | 8.5 mg |
| Croscarmellose sodium | 8.0 mg |
| Magnesium stearate | 1.00 mg |
| Colloidal Anhydrous Silica | 1.00 mg |
| Ready to make coating suspension | qs |

Imatinib mesylate and mannitol were mixed and dry granulated on a compactor. After sieving of the granulate croscarmellose sodium, magnesium stearate and colloidal anhydrous silica were added to obtain the compression mixture. After compression, the obtained tablets were optionally film coated with ready to make film coating suspension. Theoretical weight of the film coated tablets was 142 mg.

### Example 4.19

| | |
|---|---|
| Imatinib mesylate | 119.5 mg |
| Crospovidone | 8.5 mg |
| Magnesium stearate | 1.00 mg |
| Colloidal Anhydrous Silica | 1.00 mg |
| Ready to make coating suspension | qs |

Imatinib mesylate was dry granulated on a compactor. After sieving of the granulate crospovidone, magnesium stearate and colloidal anhydrous silica were added to obtain the compression mixture. After compression, the obtained tablets were optionally film coated with ready to make film coating suspension. Theoretical weight of the film coated tablets was 135 mg.

### Example 4.20

| | |
|---|---|
| Imatinib mesylate | 119.5 mg |
| Crospovidone | 16.5 mg |
| Magnesium stearate | 1.00 mg |
| Colloidal Anhydrous Silica | 1.00 mg |
| coating dispersion | qs |

Imatinib mesylate and part of crospovidone were mixed and dry granulated on a roller compactor. Obtained ribbons were milled and sieved and obtained granulate with average particle size in the range 50 to 300 µm was mixed with the rest of crospovidone, magnesium stearate and colloidal anhydrous to form a compression mixture. After compression, the obtained tablet cores were optionally film coated in perforated coating drum with ready to make film coating suspension. The weight gain of tablet cores by coating is 3.8 w/w% calculated on the weight of tablet cores. Theoretical weight of the film coated tablets was 143.2 mg.

Composition of the coating dispersion in w/w%:

| | |
|---|---|
| Kollicoat IR | 61.2 |
| Copovidone (Kollidon VA64) | 6.8 |
| Titanium dioxide | 14.0 |
| Kaolin | 16.0 |
| Sodium lauryl sulphate | 2.0 |

### Example 4.21

| | |
|---|---|
| Imatinib mesylate | 119.5 mg |
| Mannitol | 8.5 mg |
| Low-substituted hydroxypropyl cellulose LH-21 | 8.0 mg |
| Magnesium stearate | 1.00 mg |
| Colloidal Anhydrous Silica | 1.00 mg |
| Ready to make coating suspension | qs |

Imatinib mesylate and mannitol were mixed and dry granulated on a compactor. After sieving of the granulate low-substituted hydroxypropyl cellulose LH-21, magnesium stearate and colloidal anhydrous silica were added to obtain the compression mixture. After compression, the obtained tablets were optionally film coated with ready to make film coating suspension. Theoretical weight of the film coated tablets was 142 mg.

### Example 4.22

| | |
|---|---|
| Imatinib mesylate | 119.5 mg |
| Low-substituted hydroxypropyl cellulose LH-22 | 5.0 mg |
| Low-substituted hydroxypropyl cellulose LH-11 | 9.0 mg |
| Hydroxypropyl cellulose (Klucel EF) | 2.5 mg |
| Magnesium stearate | 1.00 mg |
| Colloidal Anhydrous Silica | 1.00 mg |
| Ready to make coating suspension | qs |

Imatinib mesylate and L-HPC LH-22 were mixed and dry granulated on a compactor. After sieving of the granulate low-substituted hydroxypropyl cellulose LH-11, magnesium stearate and colloidal anhydrous silica were added to obtain the compression mixture. After compression, the obtained tablets were optionally film coated with ready to make film coating suspension. Theoretical weight of the film coated tablets was 142 mg.

### Example 4.23

| | |
|---|---|
| Imatinib mesylate | 119.5 mg |
| Lactose | 8.5 mg |
| Crospovidone | 8.0 mg |
| Magnesium stearate | 1.00 mg |
| Colloidal Anhydrous Silica | 1.00 mg |
| Ready to make coating suspension | qs |

Imatinib mesylate and lactose were mixed and dry granulated on a compactor. After sieving of the granulate crospovidone, magnesium stearate and colloidal anhydrous silica were added to obtain the compression mixture. After compression, the obtained tablets were optionally film coated with ready to make film coating suspension. Theoretical weight of the film coated tablets was 142 mg.

### Example 4.24

| | |
|---|---|
| Imatinib mesylate | 119.5 mg |
| Calcium phosphate | 8.5 mg |
| Crospovidone | 8.0 mg |
| Magnesium stearate | 1.00 mg |
| Colloidal Anhydrous Silica | 1.00 mg |
| Ready to make coating suspension | qs |

Imatinib mesylate and calcium phosphate were mixed and dry granulated on a compactor. After sieving of the granulate crospovidone, magnesium stearate and colloidal anhydrous silica were added to obtain the compression mixture. After compression, the obtained tablets were optionally film coated with ready to make film coating suspension. Theoretical weight of the film coated tablets was 142 mg.

### Example 4.25

| | |
|---|---|
| Imatinib mesylate | 119.5 mg |
| Lactose | 118.0 mg |
| Starch | 30.0 mg |
| Crospovidone | 28.0 mg |
| Microcrystalline cellulose | 60.0 mg |
| Colloidal Anhydrous Silica | 1.2 mg |
| Magnesium stearate | 3.3 mg |
| Ready to make coating suspension | qs |

Imatinib mesylate, lactose, starch, crospovidone and microcrystalline cellulose were homogeneously mixed. Magnesium stearate and colloidal anhydrous silica were added to obtain the compression mixture. After compression, the obtained tablets were optionally film coated with ready to make film coating suspension. Theoretical weight of the film coated tablets was 370 mg.

### Example 4.26

| | |
|---|---|
| Imatinib mesylate | 119.5 mg |
| Lactose | 170.0 mg |
| Crospovidone | 15.0 mg |
| Microcrystalline cellulose | 50.0 mg |
| Colloidal Anhydrous Silica | 1.5 mg |
| Magnesium stearate | 4.0 mg |
| Ready to make coating suspension | qs |

Imatinib mesylate, lactose, crospovidone and microcrystalline cellulose were homogeneously mixed. Magnesium stearate and colloidal anhydrous silica were added to obtain the compression mixture. After compression, the obtained tablets were optionally film coated with ready to make film coating suspension. Theoretical weight of the film coated tablets was 370 mg.

### Example 4.27

| | **a** | **b** | **c** | **d** | **e** | **f** | **g** | **h** |
|---|---|---|---|---|---|---|---|---|
| Imatinib mesylate | 119.5 | 119.5 | 119.5 | 119.5 | 119.5 | 119.5 | 119.5 | 119.5 |
| Mannitol | 21.0 | / | / | 21.0 | 72.0 | 65.5 | / | / |
| Lactose monohydrate | / | 13.3 | 13.3 | / | / | / | 70.0 | 70.0 |
| Starch | / | 4.0 | 4.0 | / | / | / | 7.0 | 7.0 |
| Hydroxypropylcellulose | 3.0 | 3.0 | 3.0 | 2.0 | 4.5 | 4.3 | 6.8 | 6.8 |
| Purified water | q.s. | q.s. | q.s. | q.s. | q.s. | q.s. | q.s. | q.s. |
| Microcrystalline cellulose | / | 10.0 | 10.0 | 8.0 | / | / | / | / |
| Mannitol | 8.0 | / | / | / | 75.0 | 56.0 | 40.5 | 40.5 |
| Croscarmellose sodium | / | / | 6.0 | / | / | / | / | 16.0 |
| Crospovidone | 5.0 | 6.0 | / | 6.0 | 16.5 | 14.0 | 16.0 | / |
| Colloidal Anhydrous Silica | 1.0 | 1.2 | 1.2 | 1.2 | 1.0 | 0.7 | 1.2 | 1.2 |
| Magnesium stearate | 1.5 | 2.0 | 2.0 | 1.3 | 2.5 | 2.0 | 3.0 | 3.0 |
| Ready to make coating suspension | 4.0 | 4.0 | 4.0 | 4.0 | 9.0 | 8.0 | 7.0 | 7.0 |
| **Total** | 163.0 | 163.0 | 163.0 | 163.0 | 300.0 | 270.0 | 271.0 | 271.0 |

### Manufacturing process (a - h)

Imatinib mesylate was homogenized with mannitol or lactose monohydrate and starch and granulated in a fluid bed granulator with the solution of hydroxypropylcellulose in purified water.

After drying and sieving the obtained granulate was mixed with microcrystalline cellulose or mannitol, the disintegrant crospovidone or croscarmellose sodium), colloidal anhydrous silica and magnesium stearate to obtain the compression mixture. After compression, the obtained tablets were optionally film coated with ready to make film coating suspension.

Disclosed is;
1. Crystalline polymorph form II of imatinib base.
2. The crystalline polymorph according to item 1, characterized by an X-ray powder diffraction pattern exhibiting characteristic diffractions (diffraction peaks) at 5,4; 8,0; 12,6; 16,1; 17,5; 19,3; 20,7; 23,1; 24,4 and 29,1 ± 0,2 degrees 2-theta.
3. Imatinib base according to item 1 or 2, characterized by an X-ray powder diffraction pattern as in Figure 2.
4. Imatinib base according to any of items 1 to 3 characterized by having a DSC endothermic peak at 130 °C.
5. Imatinib base according to any of items 1 to 4, wherein the polymorph exhibits an infrared (IR) spectrum having characteristic peaks as shown in Figure 3.
6. Imatinib base according to any of items 1 to 5, characterized by a DSC thermogram pattern substantially as shown in Figure 4. According to one aspect, "substantially" means "having the characteristics"
7. Process for the preparation of polymorphic form II of imatinib base, comprising the steps of:
   (i) combining imatinib salt, imatinib base or reaction mixture comprising imatinib base or imatinib salts with water or with a mixture of water and alcohol C1-C4,
   (ii) precipitating of base by addition of alkaline substance to adjust the pH of suspension to a value between 7-11, preferably between 8-9, most preferably to 8.5,
   (iii) isolating the precipitated base and drying the product at temperatures between 30 °C and 120 °C, more preferably between 40 °C to 80 °C, most preferably between 50 °C to 70 °C.
8. Process for the preparation of imatinib salt, preferably imatinib mesylate, preferably at low temperatures, comprising the steps of:
   (i) combining imatinib base with water and/or mixture of water with alcohols C1-C4 and 0.9 to 1.2 equivalent of organic or inorganic acid, preferably methanesulfonic acid,
   (ii) stirring the reaction mixture at low temperatures of 5 °C to 70 °C, more preferably at 5 °C to 50 °C, most preferably at 10 °C to 30 °C until a clear solution is obtained, optionally the obtained solution is filtered before further use,
   (iii) crystallizing imatinib salt by addition of or combining with organic solvent inoculated with seeding crystals and stirring the reaction mixture, preferably until crystallization of imatinib salt, preferably imatinib mesylate form α or dimesylate is completed,
   (iv) isolating the imatinib salt, preferably polymorphic form α of imatinib mesylate and drying the product at temperatures between 30 °C and 100 °C, preferably between 50 °C and 70 °C.

   Preferably, "low temperatures" shall mean temperatures not exceeding 75 °C, preferably not exceeding 70 °C, more preferably not exceeding 60 °C.
9. Process according to item 8 wherein crystallizing imatinib salt by addition of or combining with organic solvent inoculated with seeding crystals and stirring the reaction mixture until crystallization of imatinib salt, preferably imatinib mesylate form α or dimesylate is completed is performed at temperatures between 25 and 75 °C, preferably between 30 and 70 °C and most preferably between 50 and 60 °C.
   The invention relates inter alia to the following aspects:
10. Process of conversion of any form of imatinib mesylate to imatinib mesylate form α, preferably at low temperatures, comprising the steps of:
   (i) combining imatinib mesylate with water and/or a mixture of water with alcohols C1-C4, optionally 0.01-10 %, preferably 0.01-5% molar excess of methanesulfonic acid with respect to imatinib salt can be added,
   (ii) stirring the reaction mixture at low temperatures of 5 °C to 70 °C, more preferably at 5 °C to 50 °C, most preferably at 10 °C to 30 °C until clear solution is obtained; optionally the obtained solution is filtered before further use,
   (iii) crystallizing imatinib mesylate by addition of or combining with organic solvent inoculated with imatinib mesylate α crystals and stirring the reaction mixture, preferably until crystallization of imatinib mesylate form α is completed,
   (iv) isolating the polymorphic form α of imatinib mesylate and drying the product at temperatures between 30 °C and 100 °C, preferably between 50 °C and 70 °C.

   Preferably, "low temperatures" shall mean temperatures not exceeding 75 °C, preferably not exceeding 70 °C, more preferably not exceeding 60 °C.
11. Process according to item 10 wherein crystallizing imatinib mesylate by addition of or combining with organic solvent inoculated with imatinib mesylate α crystals and stirring the reaction mixture until crystallization of imatinib mesylate form α is completed is performed at temperatures between 25 and 75 °C, preferably between 30 and 70 °C and most preferably between 50 and 60 °C.

Disclosed is:
12. Process for preparation of imatinib mesylate form α without seeding procedure and preferably at low temperatures, comprising the steps of:
   (i) combining or dissolving imatinib base and organic solvent,
   (ii) adding 0.9 to 1.2 equivalent of methanesulfonic acid or other salt formation acid, most preferably 1 to 1.05 equivalent at temperatures of 5 °C to 70 °C, more preferably 5 °C to 50 °C, most preferably 10 to 30 °C,
   (iii) stirring the solution or suspension, preferably until crystallization of imatinib salt, preferably imatinib mesylate form α is completed,
   (iv) isolating the imatinib salt, preferably polymorphic form α of imatinib mesylate and drying the product at temperatures between 30 °C and 100 °C, more preferably between 50 °C and 70 °C.

   Preferably, "low temperatures" shall mean temperatures not exceeding 75 °C, preferably not exceeding 70 °C, more preferably not exceeding 60 °C.
13. Pharmaceutical composition comprising polymorphic form II of imatinib base and a pharmaceutically acceptable carrier.
14. The pharmaceutical composition according to item 13, which is in the form of tablets, pills, powders, lozenges, sachets, soft and hard gelatine capsules or suppositories.
15. Use of polymorphic form of imatinib base, preferably polymorphic form II of imatinib base, in the preparation of a pharmaceutical composition for the treatment of a tumour disease. Polymorphic form of imatinib base, preferably polymorphic form II of imatinib base, for the treatment of a tumour disease. The definition of these diseases is known in the art.
16. Use of polymorphic form of imatinib base, polymorphic form II of imatinib base, in the preparation of a pharmaceutical composition for the treatment of gastrointestinal stromal tumours. Polymorphic form of imatinib base, preferably polymorphic form II of imatinib base for the treatment of gastrointestinal stromal tumours. The definition of these diseases is known in the art.
17. Pharmaceutical composition comprising imatinib salt obtained by a process according to any of items 8 to 12 or imatinib base obtained by a process according to item 7, and optionally a pharmaceutically acceptable carrier.
18. The pharmaceutical composition according to item 17, which is in the form of tablets, pills, powders, lozenges, sachets, soft and hard gelatine capsules or suppositories.
19. Use of an imatinib salt obtained by a process according to any of items 8 to 12 or imatinib base obtained by a process according to item 7 in the preparation of a pharmaceutical composition for the treatment of a tumour disease, preferably for the treatment of gastrointestinal stromal tumours.

## Claims

1. Process of conversion of any form of imatinib mesylate to imatinib mesylate form α, comprising the steps of:
(i) combining imatinib mesylate with water and/or a mixture of water with alcohols C1-C4, optionally 0.01-10 %, preferably 0.01-5% molar excess of methanesulfonic acid with respect to imatinib salt can be added,
(ii) stirring the reaction mixture at low temperatures of 5°C to 70°C, more preferably at 5°C to 50°C, most preferably at 10°C to 30°C until clear solution is obtained, optionally the obtained solution is filtered before further use,
(iii) crystallizing imatinib mesylate by addition of or combining with organic solvent inoculated with imatinib mesylate α crystals and stirring the reaction mixture until crystallization of imatinib mesylate form α is completed,
(iv) isolating the polymorphic form α of imatinib mesylate and drying the product at temperatures between 30°C and 100°C, preferably between 50°C and 70°C.

2. Process according to claim 1 wherein crystallizing imatinib mesylate by addition of or combining with organic solvent inoculated with imatinib mesylate α crystals and stirring the reaction mixture until crystallization of imatinib mesylate form α is completed is performed at temperatures between 25 and 75°C, preferably between 30 and 70°C and most preferably between 50 and 60°C.

3. Process for the preparation of imatinib mesylate form α comprising the steps of:
(i) combining imatinib base with water and/or mixture of water with alcohols C1-C4 and 0.9 to 1.2 equivalent of organic or inorganic acid, said organic or inorganic acid being methanesulfonic acid,
(ii) stirring the reaction mixture at low temperatures of 5°C to 70°C, more preferably at 5°C to 50°C, most preferably at 10°C to 30°C until clear solution is obtained, optionally the obtained solution is filtered before further use,
(iii) crystallizing imatinib salt by addition of or combining with organic solvent inoculated with seeding crystals and stirring the reaction mixture until crystallization of imatinib mesylate form α is completed,
(iv) isolating the polymorphic form α of imatinib mesylate and drying the product at temperatures between 30°C and 100°C, preferably between 50°C and 70°C.

4. Process according to claim 3 wherein crystallizing imatinib salt by addition of or combining with organic solvent inoculated with seeding crystals and stirring the reaction mixture until crystallization of imatinib mesylate form α is completed is performed at temperatures between 25 and 75°C, preferably between 30 and 70°C and most preferably between 50 and 60°C.

## Patentansprüche

1. Verfahren zur Umwandlung von jedweder Form von Imatinibmesylat in Imatinibmesylat-Form α, umfassend die Schritte von:
(i) Kombinieren von Imatinibmesylat mit Wasser und/oder einem Gemisch von Wasser mit C1-C4-Alkoholen, wobei gegebenenfalls ein 0,01-10 %iger, bevorzugt 0,01-5 %iger molarer Überschuss von Methansulfonsäure in Bezug auf Imatinib-Salz zugegeben werden kann,
(ii) Rühren des Reaktionsgemisches bei niedrigen Temperaturen von 5°C bis 70°C, stärker bevorzugt bei 5°C bis 50°C, am stärksten bevorzugt bei 10°C bis 30°C, bis eine klare Lösung erhalten wird, wobei die erhaltene Lösung vor weiterer Verwendung gegebenenfalls filtriert wird,
(iii) Kristallisieren von Imatinibmesylat durch Zugeben von oder Kombinieren mit einem mit Imatinibmesylat α-Kristallen angeimpften organischen Lösungsmittel und Rühren des Reaktionsgemisches, bis die Kristallisation von Imatinibmesylat-Form α vollendet ist,
(iv) Isolieren der polymorphen Form α von Imatinibmesylat und Trocknen des Produkts bei Temperaturen zwischen 30°C und 100°C, bevorzugt zwischen 50°C und 70°C.

2. Verfahren gemäß Anspruch 1, wobei das Kristallisieren von Imatinibmesylat durch Zugeben von oder Kombinieren mit einem mit Imatinibmesylat α-Kristallen angeimpften organischen Lösungsmittel und Rühren des Reaktionsgemisches, bis die Kristallisation von Imatinibmesylat-Form α vollendet ist, bei Temperaturen zwischen 25 und 75°C, bevorzugt zwischen 30 und 70°C und am stärksten bevorzugt zwischen 50 und 60°C durchgeführt wird.

3. Verfahren zur Herstellung von Imatinibmesylat-Form α, umfassend die Schritte von:
(i) Kombinieren von Imatinib-Base mit Wasser und/oder einem Gemisch von Wasser mit C1-C4-Alkoholen und 0,9 bis 1,2 Äquivalenten von organischer oder anorganischer Säure, wobei die organische oder anorganische Säure Methansulfonsäure ist,
(ii) Rühren des Reaktionsgemisches bei niedrigen Temperaturen von 5°C bis 70°C, stärker bevorzugt bei 5°C bis 50°C, am stärksten bevorzugt bei 10°C bis 30°C, bis eine klare Lösung erhalten wird, wobei die erhaltene Lösung vor weiterer Verwendung gegebenenfalls filtriert wird,
(iii) Kristallisieren von Imatinib-Salz durch Zugeben von oder Kombinieren mit einem mit Impfkristallen angeimpften organischen Lösungsmittel und Rühren des Reaktionsgemisches, bis die Kristallisation von Imatinibmesylat-Form α vollendet ist,
(iv) Isolieren der polymorphen Form α von Imatinibmesylat und Trocknen des Produkts bei Temperaturen zwischen 30°C und 100°C, bevorzugt zwischen 50°C und 70°C.

4. Verfahren gemäß Anspruch 3, wobei das Kristallisieren von Imatinib-Salz durch Zugeben von oder Kombinieren mit einem mit Impfkristallen angeimpften organischen Lösungsmittel und Rühren des Reaktionsgemisches, bis die Kristallisation von Imatinibmesylat-Form α vollendet ist, bei Temperaturen zwischen 25 und 75°C, bevorzugt zwischen 30 und 70°C und am stärksten bevorzugt zwischen 50 und 60°C durchgeführt wird.

## Revendications

1. Procédé de conversion d'une forme quelconque du mésylate d'imatinib en la forme α du mésylate d'imatinib, comprenant les étapes de :
(i) combinaison du mésylate d'imatinib avec de l'eau et/ou un mélange d'eau et d'alcools en C1-C4, le cas échéant en ajoutant un excès molaire de 0,01-10%, de préférence de 0,01-5%, d'acide méthanesulfonique par rapport au sel d'imatinib,
(ii) agitation du mélange réactionnel à faible température allant de 5°C à 70°C, de préférence de 5°C à 50°C, de manière plus préférée de 10°C à 30°C, jusqu'à obtention d'une solution limpide, le cas échéant la solution obtenue est filtrée avant utilisation ultérieure,
(iii) cristallisation du mésylate d'imatinib par addition de ou combinaison avec un solvant organique inoculé de cristaux α de mésylate d'imatinib et agitation du mélange réactionnel jusqu'à cristallisation complète de la forme α de mésylate d'imatinib,
(iv) isolement de la forme polymorphe α de mésylate d'imatinib et séchage du produit à des températures allant de 30°C à 100°C, de préférence de 50°C à 70°C.

2. Procédé selon la revendication 1, où la cristallisation du mésylate d'imatinib par addition de ou combinaison avec un solvant organique inoculé de cristaux α de mésylate d'imatinib et agitation du mélange réactionnel jusqu'à cristallisation complète de la forme α de mésylate d'imatinib, sont réalisées à des températures allant de 25 à 75°C, de préférence de 30 à 70°C, de manière plus préférée de 50 à 60°C.

3. Procédé de préparation de la forme α de mésylate d'imatinib, comprenant les étapes de :
(i) combinaison de la base imatinib avec de l'eau et/ou un mélange d'eau et d'alcools en C1-C4, et 0,9 à 1,2 équivalent d'un acide organique ou inorganique, ledit acide organique ou inorganique étant l'acide méthanesulfonique,
(ii) agitation du mélange réactionnel à faible température allant de 5°C à 70°C, de préférence de 5°C à 50°C, de manière plus préférée de 10°C à 30°C, jusqu'à obtention d'une solution limpide, le cas échéant la solution obtenue est filtrée avant utilisation ultérieure,
(iii) cristallisation du sel d'imatinib par addition de ou combinaison avec un solvant organique inoculé de cristaux d'ensemencement et agitation du mélange réactionnel jusqu'à cristallisation complète de la forme α de mésylate d'imatinib,
(iv) isolement de la forme polymorphe α de mésylate d'imatinib et séchage du produit à des températures allant de 30°C à 100°C, de préférence de 50°C à 70°C.

4. Procédé selon la revendication 3, où la cristallisation du sel d'imatinib par addition de ou combinaison avec un solvant organique inoculé de cristaux d'ensemencement et agitation du mélange réactionnel jusqu'à cristallisation complète de la forme α de mésylate d'imatinib, sont réalisées à des températures allant de 25 à 75°C, de préférence de 30 à 70°C, de manière plus préférée de 50 à 60°C.
